# EUROPEAN PATENT APPLICATION

(11) **EP 4 331 649 A1**
(43) Date of publication of application: **06.03.2024**
(21) Application number: 22193772.5
(22) Date of filing: 02.09.2022
(51) Int. Cl.: A61M 16/00, A61M 16/08, A61M 16/10, A61M 16/20, A61M 16/12

(54) **A GAS DELIVERY CONTROL SYSTEM**

(71) Applicant: Camcon Medical Limited, Cambridge, Cambridgeshire CB24 9NL (GB)
(72) Inventor: Wygnanski, Wladyslaw, Cambridge, CB24 9NL (GB); Higenbottam, Timothy, Cambridge, CB24 9NL (GB)
(74) Representative: Sharrock, Daniel John

(57) **Abstract**

A gas delivery control system (100) for controlling delivery of gas for inhalation by a recipient comprises first and second gas inlets (33,34), first and second valves (27,28) located downstream of the first and second gas inlets, respectively, first and second electromagnetic actuator assemblies (12,13) for operating the first and second valves, respectively, and a gas supply controller (7) for controlling the first and second electromagnetic actuator assemblies, wherein the gas supply controller is configured to receive a pressure signal from a pressure sensor (3), and to control the first and second electromagnetic actuator assemblies independently in response to the pressure signal. An electromagnetic actuator assembly (102) includes an armature (41) mounted in a fixed position on a shaft (60), and first and second adjustable biasing mechanisms (106,108) for applying biasing forces on the shaft, with the respective biasing forces acting in opposite axial directions.

## Description

### Field of the disclosure

The present disclosure relates to a medicinal gas delivery control system for controlling delivery of gas for inhalation by a user. It also concerns an electromagnetic actuator assembly for use in such a system.

### Background to the disclosure

Respiratory disease is an increasing global problem with wide ranging socio-economic impacts, consuming significant resources at every level. The WHO lists respiratory disease, such as Chronic Obstructive Pulmonary Disease (COPD) and Pneumonia, as the second most common cause of death. Pneumonias caused 3 million deaths per year, prior to the COVID-19 pandemic.

Death in these diseases is often a result of acute hypoxia. This is called respiratory failure (RF) where there is insufficient oxygen in the blood. It can cause death by asphyxia. Disability is also a problem in chronic RF as mobility is limited by hypoxia. In addition, patients with lung diseases such as COPD and asthma experience acute exacerbations (often due to infections) when they need to be admitted to hospital for treatment of severe hypoxia.

Respiratory failure is divided into two categories: Type 1 RF with low arterial blood oxygen (O₂) but near normal carbon dioxide (CO₂) blood levels, and Type 2 RF with low oxygen often together with high CO₂ levels.

Acute or chronic Type 1 RF is a result of significant loss of large areas of gas exchange surfaces within the lungs, through injury, inflammation or physical destruction. Type 2 RF results from weakness or loss of control of the muscles that enable breathing, and the lungs are normal. Examples include polio and muscular dystrophy.

The COVID-19 pneumonia has alerted us to the limitations of the current treatment options for Type 1 RF. This illness predominantly causes extensive damage to the alveolar septa, the surface through which gas exchange occurs in the lung. This has resulted in persistently high mortality rates during successive waves of the disease and significant oxygen shortages around the world, especially in India, during the 2021 pandemic.

Although steroid treatment (such as dexamethasone) has been shown to mitigate the severest form of the COVID-19 pneumonia, it has only blunted the high mortality rate during 2021. The mortality rate still parallels the rate of hospital admission. Large numbers of patients are still dying from asphyxia. This has focused attention on the crucial need to find new ways of restoring normoxia to patients with the severest forms of hypoxia from Type 1 RF. A new treatment is urgently needed, especially as in the next few decades it is likely that we will face frequent major outbreaks of animal to human (zoonotic) viral pneumonias.

When invasive mechanical ventilation (IMV) has been used to support oxygenation of pneumonia patients with Type 1 RF, the lungs are already structurally damaged. It has become apparent that IMV can also increase the lung damage of pneumonia and negatively impact on survival.

Non-invasive ventilation methods of supporting respiratory failure rely on delivering oxygen to patients who are able to breath on their own. Continuous positive airway pressure (CPAP) applies a sustained increased airway pressure. This "splints" the airways open and reduces the work of breathing. But it is achieved at the cost of increasing the intrathoracic pressure at levels of 8 to 25 cm H₂O. It is often used with oxygen/air flow rates of up to 25 litres/min. This requires a tight-fitting mask or helmet, but avoids intubation. The raised intra-thoracic pressure can, like IMV, cause lung damage.

An alternative approach is High Flow Nasal Oxygen (HFNO) which has also been shown to be effective in Type 1 RF in infants who also have very high peak inspiratory flow rates. However, with HFNO, oxygen is continuously flowing to the nose of a patient and most of the oxygen is wasted. This has two impacts on care: a) a lack of precision in oxygen dosing and b) an enormous waste of oxygen.

### Summary of the disclosure

The present disclosure provides a gas delivery control system for controlling delivery of gas for inhalation by a recipient, wherein the system comprises:
first and second gas inlets for coupling to respective pressurised gas supplies;
first and second valves located downstream of the first and second gas inlets, respectively;
first and second electromagnetic actuator assemblies for operating the first and second valves, respectively; and
a gas supply controller for controlling the first and second electromagnetic actuator assemblies,
wherein the gas supply controller is configured to receive a pressure signal from a pressure sensor, and to control the first and second electromagnetic actuator assemblies independently in response to the pressure signal.

The system described herein may facilitate the delivery of at least two different medicinal gases to provide respiratory support to a patient with independent control of the respective dosages. This enables a medical practitioner to deliver combinations of gases to a patient, which may be beneficial in some circumstances.

As the gas supply controller is responsive to a pressure sensor, it may control delivery of gas to a patient so that it begins at or close to the start of inspiration. This may enable the gas to be injected into the peak inspiratory flow stream of the patient, ensuring distribution to those parts of the patient's lungs that are still able to inflate and receive blood flow.

The system may enable precise dose control in a reliable and automated manner, resulting in significant resource savings in terms of clinician time and gas supplies.

In a particular example, the two gases may be oxygen and nitric oxide (NO). The control of local lung blood flow is normally regulated by endogenously released NO made in the endothelial cells that line blood vessels. In hypoxia lung diseases, the endothelial NO release is reduced causing narrowing of the pulmonary arterioles and reduced blood flow. In addition to this cause of reduced pulmonary blood flow, in COVID-19 pneumonia there is an associated pathology whereby the small pulmonary blood vessels are narrowed or blocked by constriction or by tiny blood clots.

Early COPD studies in patients with Type 1 RF showed that pulsed delivery of both oxygen and NO, daily for three months, increased exercise tolerance and also increased the exhalation of CO₂, indicating improved gas exchange. In COPD this technique has been shown to boost gas exchange by 30%. The inventors have realised that addition of exogenous NO using the present system could therefore optimise the remaining lung to take up oxygen.

While the current focus has been on COVID related pneumonia, most types of lung disease that cause Type 1 RF could benefit from this approach. The present system may extend the value of O₂-alone delivery by boosting gas exchange, revolutionising the delivery of respiratory support across multiple applications. This may primarily involve hospital-based therapies but there is also the potential to use systems according to the present disclosure in out-of-hospital applications such as domiciliary respiratory support, one of the fastest growing segments of this market.

The gas supply controller may be configured to control the first and second electromagnetic actuator assemblies so that the respective valves carry out independently timed opening and closing cycles. Preferably, the gas supply controller may be configured to control the first and second electromagnetic actuator assemblies so that the opening and closing cycles of the first and second valves start at different times.

The gas supply controller may be configured to control the first and second electromagnetic actuator assemblies so that the opening and closing cycles of the first and second valves have different durations.

The present system may facilitate precision dosing by providing control of the delivery of two gases to match patient specific peak inspiratory flow rates and by optimising gas pulse durations. The system may provide control of the opening time and closing times of the first and second valves, independently. A clinician may be able to set independent pulse durations and/or dosages per inhalation for each gas.

The system may include a pressure sensor for generating the pressure signal. The sensor may be arranged to detect a patient's breathing pattern, in particular the start of each inhalation. This signal may then be used by the gas supply controller to determine when to trigger delivery of gas pulses to the patient. The sensitivity of the sensor may be adjustable.

The pressure signal from the pressure sensor may be dependent on or indicative of the gas pressure within a mask worn by a patient or in a nasal passage of a patient, for example. This may enable the system to determine the timing of the onset of each inhalation and the rate of a patient's breathing. In Type 1 RF patients breathing rates can increase from some 15 bpm to over 40 bpm. The time available for inhaling is markedly reduced, requiring a steep increase in the peak inspiratory flow rate with each breath. This peak inspiratory flow rate occurs shortly after the start of inspiration and then tapers off during the inhalation.

This early inspiratory phase of inspiration is critically important for gas exchange, as it is when the major expansion of the lungs begins and the inhaled air is drawn into the lungs travelling first to the most compliant parts of the lungs and where there is the lowest airway resistance. The present system may enable precise control of the timing of the delivery of a bolus of gas so that it is provided to the patient at the early part of the inspiration and likely to be entrained into the best ventilated parts of the lungs.

Preferred examples of the present system may include first and second flow rate control devices downstream of the first and second gas inlets, respectively. The gas supply controller may be configured to control the first and second flow rate control devices.

Regulation of the delivery flow rates may be used to match the peak inspiratory flow rate of a patient. By so doing it is possible to entrain the delivered gas pulses into remaining well-ventilated regions of the patient's lungs and avoid dilution of the gases by ambient air.

Pulse durations and/or gas flow rates may be individually adjusted. This adjustment may be manual and/or semi-automated with breath pattern prediction by the gas supply controller in response to the pressure sensor, peak flow estimation and/or a closed loop control system. Using the present system, the gases from two supplies may be delivered simultaneously, in a partially overlapping manner or consecutively as appropriate.

Preferably, the first and second flow rate control devices are located downstream of the first and second valves, respectively. This may serve to reduce the likelihood of sudden pressure increases caused by rapid release of compressed gas, particularly where tubes having a degree of elasticity are employed.

The gas supply controller may be configured to receive an oxygen level signal from an oximeter. This may facilitate closed loop control by the gas supply controller of the delivery of the gases (that is, the pulse start times, durations and/or flow rates) with reference to target values for the level of oxygen in a patient's blood. Also, an alarm may be triggered by the controller when a predetermined oxygen level threshold is exceeded.

The system may include first and second flow smoothing assemblies downstream of the first and second gas inlets and upstream of the first and second valves, respectively. Such assemblies may reduce the risk of rapid pressure drop in a gas supply from being transmitted into the system.

The system may include first and second flow amplification devices downstream of the first and second gas inlets and upstream of the first and second valves, respectively, for producing an instantaneous gas flow rate higher than a flow rate of a pressurised gas supply coupled to the respective gas inlet. This may be beneficial when the available gas supply only provides a relatively low flow rate. For example, each flow amplification device may comprise a resilient container.

A mixing device may be included in the system which has a single outlet, wherein the mixing device is configured to combine gases received via the first and second valves and output the combined gases from its single outlet.

The system may include a warming device for raising the temperature of a gas flow in the system. Preferably, the warming device is located downstream of the mixing device. The warming device may be configured to transfer heat energy from its ambient surroundings to a gas flow in the system. Where gases inputted into the system are below room temperature due to rapid decompression, such a warming device may act to raise the temperature of the gases to make inspiration of the gases more comfortable for a patient.

The present disclosure also provides an electromagnetic actuator assembly comprising:
an armature mounted in a fixed position on a shaft, wherein the armature comprises a permanent magnet, and the shaft extends along a central axis of the actuator assembly;
two electrically conductive coils; and
two coil cores, each of which extends within a respective coil,
wherein the armature and shaft are movable relative to the coils between first and second stable rest positions by passing a current through at least one of the coils, and
in each rest position, the armature is closer to one of the coil cores than the other of the coil cores and is spaced by a gap from the closer coil core,
and wherein the actuator assembly includes first and second adjustable biasing mechanisms for applying biasing forces on the shaft, with the respective biasing forces acting in opposite axial directions.

The adjustable biasing mechanisms may be used to adjust the mechanical forces acting on the shaft of the actuator assembly. This may enable compensation for variation in the magnetic and/or mechanical forces acting on the shaft of the actuator assembly.

The adjustable biasing mechanisms may counter magnetic attraction forces acting on the armature and ensure that equilibrium conditions defining a rest position are reliably achieved before the armature comes into physical contact with an adjacent coil core. Accordingly, the biasing mechanisms may be adjusted such that this contact does not occur and any associated clicking noises are avoided. The biasing mechanisms may be adjusted such that the mechanical forces exerted on the actuator shaft are substantially equal to the magnetic attraction forces (due to attraction of the armature to an adjacent coil core) acting on it in each stable rest position.

Each biasing mechanism may comprise a resilient component having (a) one end arranged to exert a force on the shaft and (b) an opposite end, with the location of the opposite end relative to the actuator housing being dictated by an adjustable stop member. The position of each adjustable stop member relative to the actuator housing may be altered manually. This adjustment may be carried out (and the stop members then fixed in position) during the production and testing of the actuator assembly.

The actuator assembly may include a valve assembly coupled to the shaft such that, when the shaft is in its first stable rest position, the valve assembly is in an open configuration, and when the shaft is in its second stable rest position, the valve assembly is in a closed configuration. Preferably, the valve assembly includes a sleeve mounted around the shaft, and the sleeve closes a flow path through the valve assembly when the valve assembly is in its closed configuration, and defines part of the flow path through the valve assembly when the valve assembly is in its open configuration.

The sleeve may include a first portion having a first diameter and defining an outer surface which closes the flow path through the valve when the valve assembly is in its closed configuration. Furthermore, the sleeve may include a second portion having a second diameter which is less than the first diameter, wherein the second portion defines part of the flow path through the valve assembly when the valve assembly is in its open configuration.

In a preferred implementation, the sleeve is slidably mounted on the shaft so that the sleeve is able to slide relative to the shaft in an axial direction. The sleeve may be slidable to a limited extent relative to the shaft. The ability of the sleeve to slide relative to the shaft may provide a degree of tolerance in the dimensions of the valve assembly to increase its reliability and durability.

The valve assembly preferably includes a valve body which defines a conduit, wherein the sleeve is located within the conduit for reciprocating movement along the conduit, and the conduit and sleeve are dimensioned such that there is room for the sleeve to move laterally relative to the conduit to provide a degree of tolerance in the lateral location of the sleeve within the conduit. This enables the valve assembly to provide some tolerance in the alignment of the shaft relative to the valve body, and avoids excessive friction between the sleeve and the valve body.

The actuator assembly may include an emergency manually operable control located outside the actuator housing and mechanically coupled to the shaft (or forming part of the shaft) to enable a user to exert an axial force on the shaft to urge it towards one of its stable rest positions. This may provide a manual safety reset button so that a user may force the respective valve into an open or closed configuration.

### Brief description of the drawings

Examples of the present disclosure will now be described with reference to the accompanying schematic drawings, wherein:
Figure 1 is a block diagram of a gas delivery control system according to the present disclosure;
Figures 2 and 3 are cross-sectional views of an electromagnetic actuator and valve assembly according to the present disclosure, with the valve in open and closed configurations, respectively; and
Figure 4 is an enlarged cross-sectional view of part of the electromagnetic actuator and valve assembly shown in Figures 2 and 3, with the valve in its closed configuration.

### Detailed description

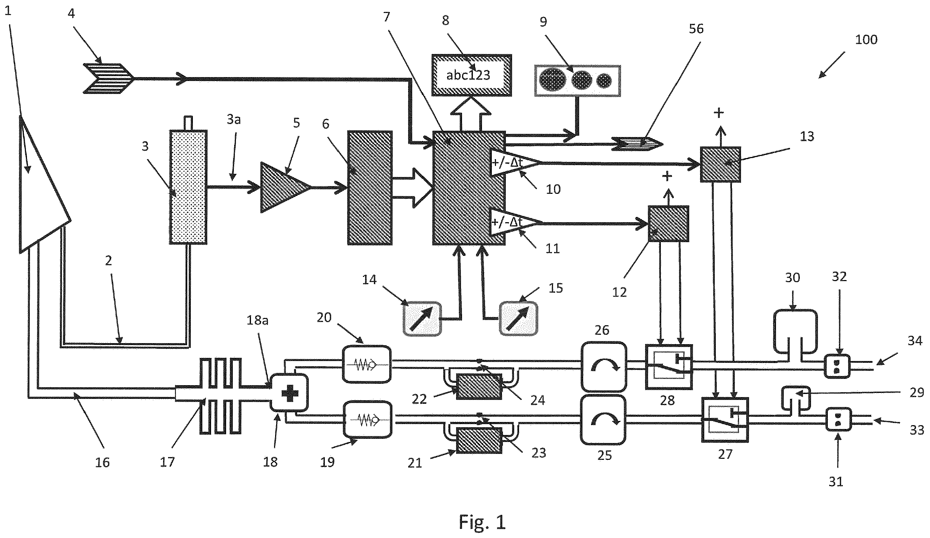

Figure 1 shows a gas delivery control system 100 according to an example of the present disclosure. The system is shown in combination with a face mask 1 for use by a patient. Preferably, a nasal cannula may be used to deliver the gas to the nose of the patient.

The system includes first and second gas inlets 33, 34 for coupling to respective supplies of pressurised gas. Each gas inlet is coupled to a respective flow restrictor 31, 32 and resilient container 29, 30. Each flow restrictor and container in combination acts as a passive pneumatic lowpass filter to inhibit unwanted pulsations in a gas supply network from travelling further into the system.

Each resilient container may be used as a rapid source of gas in a flow amplification procedure. Each container may be arranged to inflate and store gas from the associated supply when the respective downstream valve is closed. These containers may act as pneumatic capacitors, able to briefly provide a high gas flow rate, significantly greater than that available from the gas source. This may be beneficial in hospital wards where oxygen supply may be restricted to 10 litres/min or in care homes where oxygen may be delivered from mechanical trade typically able to supply only 5 litres/min, for example. The resilient containers may facilitate pulsed gas delivery at peak flow rates of up to 50 litres/min or more, even with these low flow rate supplies.

Each gas flow is then fed to a respective valve 27, 28 which operates to control the dosage of each gas. A pneumatic flow restrictor (which may include a flow indicator) 25, 26 is provided downstream of each valve. Each flow restrictor is operable manually and/or by the controller 7 to control the flow rate of the associated gas.

Each gas is then carried to a fast-responding dynamic flow meter 21, 22 which is responsive to the pressure difference across a calibrated flow restrictor 23, 24 connected in parallel with the associated flow meter. Each flow meter measures the gas flow rate in the associated flow path and outputs a signal to the controller 7 which is responsive to or indicative of the measured gas flow rate.

Subsequently, each gas flows to a respective check valve 19, 20 which prevents interference between the individual gas flows. Downstream of the check valves, the gas flows are combined in a mixing chamber 18 having a single outlet 18a.

The outlet 18a of the mixing chamber is fluidically coupled to a warm-up chamber 17. This chamber is provided to raise the temperature of the gas flow if needed by facilitating the transfer of heat energy from the ambient surroundings of the chamber to the gas flow through the chamber. The chamber may be configured to provide a relatively large surface area between the ambient surroundings and the gas flow (in comparison with a straight cylindrical tube of the same length) to enhance the transfer of heat energy. For example, the chamber may have ribbed walls. Gas flowing out of the chamber passes into a breathing tube 16 which is coupled to the face mask 1.

A pressure sensing tube 2 is also fluidically coupled to the face mask 1. The diameter of the pressure sensing tube 2 may be less than that of the breathing tube 16. The pressure sensing tube is coupled to a high sensitivity differential pressure sensor 3. The pressure sensor is operable to convert gas pressure fluctuations within the pressure sensing tube 2 into an electrical analogue signal which is outputted along line 3a. The pressure sensor may be in the form of a symmetrical resistive Wheatstone bridge, for example.

The electrical signal carried along line 3a is filtered and amplified by an analogue active lowpass filter 5. The output signal from filter 5 is converted into a digital form by an analogue-to-digital converter 6.

The output signal from the analogue-to-digital converter 6 is fed to a gas supply controller 7 which may comprise a microprocessor. The controller has an input port 4 for connection to an oximeter, such as a pulse oximeter manufactured by Nonin Medical, Inc., for example.

Two pulse duration or gas dose controllers 14, 15 are also communicatively coupled to the controller 7 for facilitating independent adjustment by a user of the delivery to a patient of respective gases inputted into the system via the inlets 33, 34. Each controller may enable adjustment of two parameters, namely the pulse duration and the gas flow rate, to control the amount of gas delivered in each pulse.

A display screen 8 is coupled to the gas supply controller 7 for presenting data to a user. The screen may be provided in combination with an input device or it may be touch-sensitive to facilitate control of the system by a user. The screen may also be used to display operating and alarm conditions. The system may also include a device for emitting a sonic warning signal when an alarm condition is detected.

The system may include a status display 9 for giving an indication of a current system parameter. For example, the status display may include a set of three coloured lights to indicate the current oxygen level detected by an oximeter coupled to port 4 of the system. In one implementation, a green light may indicate that the oxygen level is within a desired range, a red light may be illuminated when the oxygen level is too low, and an orange light illuminated when the oxygen level is too high.

The system includes an output port 56 coupled to the controller 7 for outputting data to another device and/or receiving data. For example, the output port may be arranged to facilitate wireless data communication, such as via a Bluetooth connection, which may be with a local data network or an individual user's receiver.

The gas supply controller 7 is communicatively coupled to two electromagnetic actuator assemblies 12, 13. Each actuator assembly is arranged to operate a respective valve 28, 27. The controller is arranged to output control pulses to the actuator assemblies. The controller may include pulse start time adjusters 10, 11 for controlling the start time of each gas pulse.

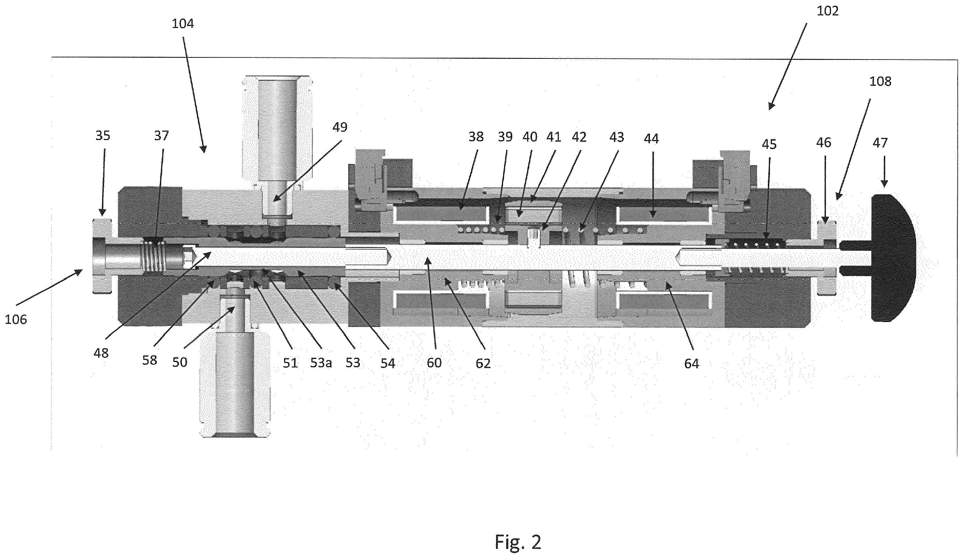

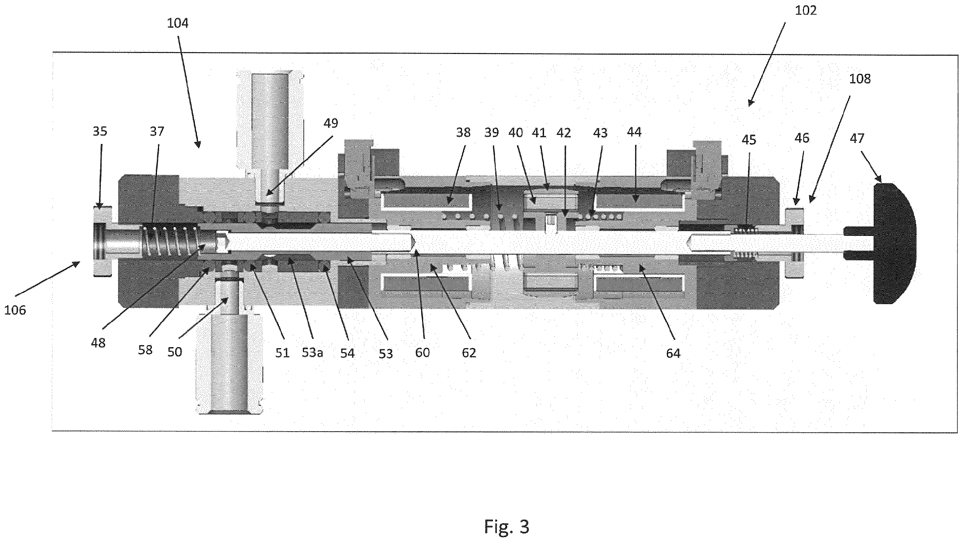

Preferably, each electromagnetic actuator is a bistable device switchable between its stable positions by an input pulse. A suitable actuator configuration shown in Figures 2 and 3.

The gas supply controller 7 is configured to detect a patient's breathing pattern in response to the signals received from the analogue-to-digital converter 6. This is then used to determine an optimal time for starting delivery of each gas with reference to the predicted start time of each inhalation by the patient. The duration of each pulse may be controlled by the controller with reference to signals received from the oxygen level input port 4. The size of each dose may be monitored by the controller with reference to output signals received from the flow meters 21, 22.

Figures 2 and 3 show an electromagnetic actuator and valve assembly 102 according to an example of the present disclosure, with the valve in its open and closed positions, respectively.

The actuator includes a slidably mounted central shaft 60. The shaft may consist of a single element or may be formed from multiple parts which are rigidly fixed together. An armature 41 is mounted on the shaft and includes an annular permanent magnet 40 which extends around the shaft. The magnet is mechanically coupled to the shaft by a mount 42.

The actuator assembly includes a pair of cylindrical coils 38, 44 which are coaxially mounted around the shaft on either side of the armature. Each coil extends around a respective coil core 62, 64. A pair of resilient elements in the form of compression springs 39 and 43 are located between a respective side of the armature and one of the coil cores 62, 64. The actuator assembly may be switchable between two stable rest positions by application of a suitable current pulse to one or both of the coils. In each rest position, the armature is closer to one of the coil cores than the other of the coil cores and is spaced by a gap from the coil core due to a force exerted on the armature by compression of one of the springs 39, 43. An actuator assembly with such a configuration is described for example in International Patent Publication No. WO2018/046909 filed by the present applicant.

The actuator and valve assembly 102 includes a valve assembly 104 located at one end of its actuator. The shaft 60 extends longitudinally through the valve assembly. The valve has an inlet port 50 and an outlet port 49. A sleeve 53 is located around the shaft which includes a central portion 53a having a reduced diameter.

When the actuator is in the stable rest position depicted in Figure 2, the inlet port 50 of the valve is fluidically coupled to the outlet port 49 via a space defined between the reduced diameter portion 53a of the sleeve 53 and the body of the valve. In this configuration, O-rings 58 and 54 form a gas-tight seal between the sleeve 53 and the body of the valve.

When the actuator is in the stable rest position depicted in Figure 3, the inlet port 50 of the valve is closed by the sleeve 53. In this configuration, O-rings 58 and 51 form a gas-tight seal between the sleeve and the body of the valve.

The actuator and valve assembly 102 includes first and second adjustable biasing mechanisms 106, 108 for applying biasing forces on the shaft. The biasing mechanism 106 comprises a resilient component in the form of a compression spring 37 and an adjustable stop member 35. The biasing mechanism 108 comprises a resilient component in the form of a compression spring 45 and an adjustable stop member 46. Each stop member defines a circumferential screw thread which is engaged with a complimentary thread defined by the actuator body so that rotation of each stop alters its axial location relative to the actuator body. The stop member 35 is spaced from one end 48 of the shaft 60. The other stop member 46 has an annular configuration and the shaft extends through the central opening of the stop member.

The biasing mechanisms exert adjustable biasing forces on the shaft in opposite directions to each other. This configuration facilitates adjustment of the operational properties of the actuator. This may ensure quiet operation of the actuator, that is, by preventing the armature 40 from coming into physical contact with either of the coil cores 62, 64.

The biasing mechanisms of the actuator assembly may be adjusted to ensure reliable selection of, and retention in, each of its stable rest positions by acting on the shaft such that equilibrium conditions are reached in each stable rest position. Such adjustment may be desirable to compensate for any variations in the mechanical and/or magnetic properties of an individual actuator assembly.

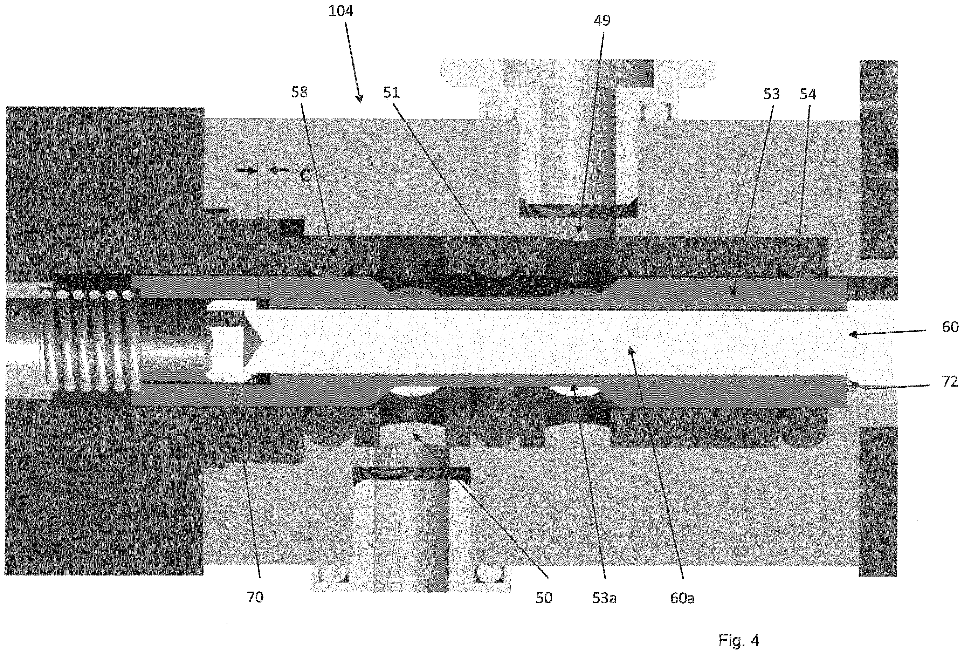

Figure 4 shows an enlarged view of part of the valve assembly shown in its open configuration in Figure 3. This is to illustrate how, in preferred examples, the sleeve 53 is able to slide relative to the shaft 60 to a limited extent, along the axial direction. The sleeve surrounds a portion 60a of the shaft which has a reduced diameter. The shaft defines shoulders 70 and 72 at the axially outer and inner ends of the portion 60a, respectively, which are able to exert axial forces on the sleeve in opposite directions. A radially inner portion of the sleeve 53 which extends between the shoulders 70 and 72 is slightly shorter in the longitudinal direction than portion 60a so that there is a longitudinal clearance "C" defined therebetween, allowing a degree of freedom or "floating" in the longitudinal position of the sleeve relative to the shaft.

To enable the sleeve to slide freely relative to the shaft, the diameter of portion 60a of the shaft is slightly less than the diameter of the surrounding opening in the sleeve. Similarly, the maximum diameter of the sleeve is preferably slightly less than that of the surrounding conduit defined by the valve body to provide a degree of freedom in the lateral location of the sleeve within the conduit. This enables the sleeve to slide freely relative to the valve body and avoids excessive friction between the sleeve and the surrounding walls of the valve body.

This configuration ensures fast and equal valve transitions in both directions, with increased tolerance of dimensional variations.

As the sleeve is able to slide to a limited extent relative to the shaft and move laterally relative to the valve body, the degree to which the sleeve rubs against the O-rings 51, 54 and 58 is reduced, thereby extending the durability of the valve.

Where the actuator and valve assembly 102 is used to control oxygen delivery, the system may be configured to switch to a default mode of constant oxygen supply with the relevant valve open. This may occur in emergency circumstances or when a patient's breathing pattern cannot be detected by the controller 7.

An emergency button or knob 47 is rigidly mounted onto one end of the shaft 60. This enables a user to manually move the actuator into one of its stable rest positions to force the associated valve into an open or closed configuration. This may be used in the event of a loss of power, for example.

In one implementation, a gas delivery control system according to the present disclosure may be arranged to deliver combined gas flow rates in a range between 2 and 75 litres/min, with the volume of the gas dosage per inhalation to be in a range from 10 to 300 millilitres for example, and with the delivery of each gas dose to be achieved within around 10 to 300ms of the start of each inspiration.

It will be appreciated that, whilst the system depicted in Figure 1 is configured to control the delivery of gas from two supplies, other systems in accordance with the present disclosure may be arranged to control the delivery of gas from more than two supplies to a patient. A separate gas inlet, valve and electromagnetic actuator assembly would be provided in association with each gas supply.

## Claims

1. A gas delivery control system for controlling delivery of gas for inhalation by a recipient, wherein the system comprises:
first and second gas inlets for coupling to respective pressurised gas supplies;
first and second valves located downstream of the first and second gas inlets, respectively;
first and second electromagnetic actuator assemblies for operating the first and second valves, respectively; and
a gas supply controller for controlling the first and second electromagnetic actuator assemblies,
wherein the gas supply controller is configured to receive a pressure signal from a pressure sensor, and to control the first and second electromagnetic actuator assemblies independently in response to the pressure signal.

2. A system of claim 1, wherein the gas supply controller is configured to control the first and second electromagnetic actuator assemblies so that the respective valves carry out independent opening and closing cycles, and preferably the gas supply controller is configured to control the first and second electromagnetic actuator assemblies so that the opening and closing cycles of the first and second valves start at different times.

3. A system of claim 1 or claim 2, wherein the gas supply controller is configured to control the first and second electromagnetic actuator assemblies so that the opening and closing cycles of the first and second valves have different durations.

4. A system of any preceding claim, including first and second flow rate control devices downstream of the first and second gas inlets, respectively, and preferably the first and second flow rate control devices are downstream of the first and second valves, respectively.

5. A system of claim 4, wherein the gas supply controller is configured to control the first and second flow rate control devices.

6. A system of any preceding claim, wherein the gas supply controller is configured to receive an oxygen level signal from an oximeter.

7. A system of any preceding claim, including first and second flow smoothing assemblies downstream of the first and second gas inlets and upstream of the first and second valves, respectively.

8. A system of any preceding claim, including first and second flow amplification devices downstream of the first and second gas inlets and upstream of the first and second valves, respectively, for producing an instantaneous gas flow rate higher than a flow rate of a pressurised gas supply coupled to the respective gas inlet, and preferably each flow amplification device comprises a resilient container.

9. A system of any preceding claim, including a mixing device having a single outlet, wherein the mixing device is configured to combine gases outputted by the first and second valves and to output the combined gases from its single outlet.

10. A system of any preceding claim, including a warming device for raising the temperature of a gas flow in the system, wherein preferably the warming device is downstream of the mixing device, and preferably the warming device is configured to transfer heat energy from its ambient surroundings to a gas flow in the system.

11. An electromagnetic actuator assembly comprising:
an armature mounted in a fixed position on a shaft, wherein the armature comprises a permanent magnet, and the shaft extends along a central axis of the actuator assembly;
two electrically conductive coils; and
two coil cores, each of which extends within a respective coil,
wherein the armature and shaft are movable relative to the coils between first and second stable rest positions by passing a current through at least one of the coils, and
in each rest position, the armature is closer to one of the coil cores than the other of the coil cores and is spaced by a gap from the closer coil core,
and wherein the actuator assembly includes first and second adjustable biasing mechanisms for applying biasing forces on the shaft, with the respective biasing forces acting in opposite axial directions.

12. An actuator assembly of claim 11 including an actuator housing, wherein each biasing mechanism comprises a resilient component having (a) one end arranged to exert a force on the shaft and (b) an opposite end, with the location of the opposite end relative to the actuator housing being dictated by an adjustable stop member.

13. An actuator assembly of claim 11 or claim 12, including a valve assembly coupled to the shaft such that, when the shaft is in its first stable rest position, the valve assembly is in an open configuration, and when the shaft is in its second stable rest position, the valve assembly is in a closed configuration, wherein preferably the valve assembly includes a sleeve mounted around the shaft, and the sleeve closes a flow path through the valve assembly when the valve assembly is in its closed configuration, and defines part of the flow path through the valve assembly when the valve assembly is in its open configuration, preferably the sleeve is slidably mounted on the shaft so that the sleeve is able to slide relative to the shaft in an axial direction, and preferably the valve assembly includes a valve body which defines a conduit, the sleeve is located within the conduit for movement along the conduit, and the conduit and sleeve are dimensioned such that there is room for the sleeve to move laterally relative to the conduit to provide a degree of tolerance in the lateral location of the sleeve within the conduit.

14. An actuator assembly of any of claims 11 to 13 including an emergency button located outside the actuator housing and mechanically coupled to the shaft to enable a user to exert an axial force on the shaft to urge it towards one of its stable rest positions.

15. A system of any of claims 1 to 10, wherein the first and second electromagnetic actuator assemblies are configured according to any one of claims 11 to 14.
